# EUROPEAN PATENT APPLICATION

(11) **EP 3 388 033 A1**
(43) Date of publication of application: **17.10.2018**
(21) Application number: 17165929.5
(22) Date of filing: 11.04.2017
(51) Int. Cl.: A61F 2/95

(54) **INTRAVASCULAR DEVICE HAVING EXPANDABLE RADIAL EXTENSION SUPPORTED BY INTRAVASCULAR FLUID PRESSURE COMPENSATION**

(71) Applicant: Koninklijke Philips N.V., 5656 AE Eindhoven (NL)
(72) Inventor: HAKKENS, Franciscus Johannes Gerardus, 5656 AE Eindhoven (NL); HENDRIKS, Cornelis Petrus, 5656 AE Eindhoven (NL); JOHNSON, Mark Thomas, 5656 AE Eindhoven (NL)
(74) Representative: de Haan, Poul Erik

(57) **Abstract**

An intravascular device (200) comprising an active member (202) for reversibly expanding a radial extension of the intravascular device inside a luminal extension of the vessel and comprising a reversibly deformable active membrane(206) that is arranged and configured to change its shape against a variable intravascular fluid pressure exerted on the active membrane in an inserted state by an intravascular fluid, an intravascular pressure compensation cavity (208), which is fluidicly sealed from the intravascular fluid and which includes a first chamber (208.1) that is covered by the active membrane (206), and a second chamber (208.2) that is covered by a compensation membrane (210), which is reversibly deformable in response to the variable intravascular fluid pressure, wherein the first chamber (208.1) and the second chamber (208.2) are in pressure communication with each other and, via the active membrane (206) and the compensation membrane (210), with the intravascular fluid.

## Description

### FIELD OF THE INVENTION

The present invention relates to an intravascular device for insertion into a vessel of a living being and to an intravascular system comprising such an intravascular device.

### BACKGROUND OF THE INVENTION

US 2005/0165439 A1 describes medical devices for implantation or insertion into a body lumen, for example, catheters, guidewires, stents and aneurysm coils. The devices comprise electrically actuated materials, such as electroactive polymers and piezoelectric and electrostrictive materials, which enhance or expand their functionality. One device disclosed in this document is a balloon catheter, which can be used, for example, to expand a stent at a site of arterial obstruction.

### SUMMARY OF THE INVENTION

Known devices of this kind suffer in accuracy and stability of shape in an expanded state due to variations in intravascular fluid pressure acting against a force driving the expansion.

It is an object of the present invention to provide an intravascular device that allows improving accuracy and shape stability of expansion in spite of variations in intravascular fluid pressure.

According to the present invention an intravascular device for insertion into a vessel of a living being is provided, which comprises:
- an active member for reversibly expanding a radial extension of the intravascular device inside a luminal extension of the vessel and comprising a reversibly deformable active membrane that is arranged and configured to change its shape against a variable intravascular fluid pressure exerted on the active membrane in an inserted state by an intravascular fluid;
- an intravascular pressure compensation cavity, which is fluidicly sealed from the intravascular fluid and which includes
- a first chamber that is covered by the active membrane, and
- a second chamber that is covered by a compensation membrane, which is reversibly deformable in response to the variable intravascular fluid pressure, wherein
- the first chamber and the second chamber are in pressure communication with each other and, via the active membrane and the compensation membrane, with the intravascular fluid.

The intravascular device of the present invention comprises an active member for reversibly expanding a radial extension of the intravascular device and an intravascular pressure compensation structure in the form of an intravascular pressure compensation cavity. Expansion of the radial extension refers to an expansion in any direction having a component pointing in a direction perpendicular to a current or most recent insertion movement, which may correspond a longitudinal direction of the intravascular device.

It thus provides a solution for accurate in-body use of the intravascular device, i.e., an accurate operation of the active member when the intravascular device is positioned inside a luminal extension of a vessel of a living being. The operation accuracy is improved in that the pressure compensation structure ensures that the reversible expansion of the radial extension of the intravascular device using the active membrane does not vary with a variation in intravascular fluid pressure. The intravascular fluid pressure is thus also acting on the inner side of the active membrane. The shape of the active membrane of the active member is stabilized by the pressure compensation cavity, thus preventing a large shape fluctuation as a result of intravascular fluid pressure variation. Also, the force generated for expanding the radial extension of the intravascular device against the intravascular fluid pressure is increased.

In the example of a blood vessel, fluid pressure variations occur as blood pressure variations for instance between systolic and diastolic pressure extrema during the heart cycle. For example, a blood pressure variation between 80 and 120 mmHg (that is between 10666 and 15999 Pa) involves a pressure variation of more than 5000 Pa. The pressure compensation solution provided by the intravascular device of the present invention avoids a shape fluctuation resulting from this pressure variation, which is beneficial for a wide range of in-body applications, strongly increasing stroke, accuracy and stable operation of the active member.

Furthermore, the intravascular device of the present invention allows strongly increasing an obtainable expansion of the radial extension in comparison with an otherwise identical intravascular device that does not have the pressure compensation cavity. Actuating against the blood pressure strongly limits a maximum expansion that can be obtained. For instance, a time average of the blood pressure of 100 mmHg means that a pressure of 13332 Pa is acting against the active member, which is a very significant pressure amount. However, with the pressure compensation structure of the intravascular device of the present invention this pressure amount is compensated, and can in some embodiments even be overcompensated, thus further increasing the available range of expansion or force generated by the active member.

The description now turns to embodiments of the intravascular device of the present invention.

There are different possibilities for the mutual arrangement of the first and second chamber of the intravascular pressure compensation cavity. In some embodiments, the first chamber and the second chamber are arranged at a longitudinal distance from each other along a longitudinal extension of the intravascular device. In such embodiments, the pressure compensation cavity further comprises a connection cavity that extends from the first chamber to the second chamber and establishes the pressure communication between the first and the second chamber.

In other embodiments, in particular in embodiments that do not require a symmetrical shape of the active member when expanding the radial extension of the intravascular device, the first chamber and the second chamber can be arranged at the same longitudinal position with respect to the longitudinal extension of the intravascular device, or with an overlap of longitudinal extension. In such embodiments, assuming a roughly cylindrical shape of the intravascular device, the first and second chambers thus may be arranged at different circumferential positions, suitably also in mutual pressure communication via a connection cavity.

For some application cases, embodiments of the intravascular device comprise a central lumen enclosed by a wall. This is advantageous for many purposes, such as for guiding surgical or sensing instrumentation to a site of interest along the longitudinal extension of the intravascular device after insertion, in particular to a site near the active member. In such embodiments, the intravascular pressure compensation cavity is arranged in the wall of the intravascular device. In order to achieve the pressure compensation of the present invention, the pressure compensation cavity is in embodiments of this kind not in pressure communication with the central lumen. This can be achieved by suitable sealing.

In some of these embodiments that are used for achieving optical sensing using instrumentation guided through the central lumen, the wall of the intravascular device is configured to allow transmission of electromagnetic radiation in a predetermined spectral sensing range. The extension of the longitudinal range of the wall allowing transmission of electromagnetic radiation is suitably limited to a desired window range. In different variants of these embodiments, an optical sensor is arranged in a predetermined longitudinal position or can be shifted along the longitudinal extension of the central lumen to be positioned in a variable position. Suitably, the optical sensor is rotatable inside the central lumen relative to the enclosing wall, thus allowing rotation coverage of a large field of view extending over a large or even the full circumferential range of the intravascular device. The optical sensor is in some embodiments configured to emit sensor light in the spectral sensing range through the wall and receive sensor light that has been scattered by an ambient of the intravascular device, for instance by intravascular fluid such as blood in the vessel, by a wall of the vessel, or by plaque deposited inside the vessel. In some embodiments, the optical sensor includes a camera.

For other application cases, however, the intravascular device has no central lumen. Embodiments of this kind provide additional design options, in particular more flexibility for arranging the pressure compensation cavity. For instance, embodiments of intravascular guidewires do not have a central lumen.

Pressure communication between the intravascular fluid and different structural entities of the intravascular device means that the pressure exerted by the intravascular fluid is passed on to the different entities. To this end, for instance, the active compensation membrane is reversibly deformable in response to the variable intravascular fluid pressure. Suitable implementations of the active and compensation membranes include a thin flexible polymer sheath. The membranes can for instance be made of thermoplastic polymers, such as a polyether block amide, for instance known under the trade name Pebax. Other suitable materials include polyethylene terephthalate (PET), polyurethane, silicones, blends of the mentioned materials, multilayer structures made of the mentioned materials. Pressure communication between the first and second chamber of the pressure compensation cavity can be achieved by any suitable medium that allows pressure communication. Different embodiments, thus, have the pressure compensation cavity filled with different media for achieving pressure communication. Suitable media are in particular a gas, a liquid or a viscoelastic material. The use of an air filling is currently preferred for ease of manufacturing.

For protection of the compensation membrane from mechanical damage, some embodiments comprise a stiff protective cover piece that is arranged between the compensation membrane and the intravascular fluid and that contains at least one opening for allowing pressure communication between the intravascular fluid and the compensation membrane.

The active member can be implemented in different ways. In some embodiments, it comprises an actuator that is configured to receive a first control signal and to drive an expansion or contraction of the active membrane in accordance with the received first control signal. The first control signal can be provided by different forms of signal communication, be it an electrical signal, a magnetic signal, an electromagnetic signal, such as a visible or infrared light signal or a radio frequency signal.

Different options exist for implementing the actuator. Suitable actuators comprise a shape memory alloy for actuating the active member, or a piezo motor, for instance using a ceramic piezoelectric material. In advantageous embodiments of the intravascular device, the actuator comprises an electroactive polymer layer that is in mechanical contact with the active membrane and that is configured to controllably change its layer shape in accordance with the received first control signal. Electro active polymers (EAPs) are an emerging class of materials within the field of responsive materials. EAPs can easily be manufactured into various shapes allowing easy and flexible integration into different embodiments of the intravascular device. Advantages of EAPs include a small form factor and a low-power, noiseless, accurate, high resolution operation with fast and reversible response.

Electro active polymers suitable for use in the intravascular device of the present invention can be subdivided in field-driven and ionic-driven materials. Field driven EAPs are actuated by an electric field through direct electromechanical coupling, while the actuation mechanism for ionic EAPs involves the diffusion of ions. Examples of field driven EAPs are electrostrictive polymers such as dielectric elastomers, PVDF (polyvinylidenfluoride) based relaxor polymers and liquid crystal elastomers (LCE). Examples of ionic driven EAPs are conjugated polymers, carbon nanotube-polymer composites and ionic polymer metal composites (IPMC).

The active membrane of some embodiments has a ring shape, in particular in the form of a circumferential ring forming part of a surface of the intravascular device. The active membrane may for instance be arranged in a wall of an outer sheath of the intravascular device.

In order to further enhance expansion and generation of force by the active membrane against the intravascular fluid pressure, the active member of some embodiments of the intravascular device further comprises a compensation actuator that is configured to receive a compensation control signal and to drive a deformation of the compensation membrane in accordance with the received compensation control signal. In such embodiments, the compensation actuator for instance comprises a (second) electroactive polymer layer that is in mechanical contact with the compensation membrane and that is configured to controllably change its layer shape in accordance with the compensation control signal, similar to the first EAP layer that is used in embodiments for actuation of the active membrane.

In a particularly advantageous group of embodiments, additional use can be made of the active member for reduction of consumption of a blood substitute such as saline or contrast fluid in optical sensing or imaging applications. Blood strongly scatters light. Therefore, spectral analyses, for instance of a blood vessel wall, of plaque in a vessel etc. or optical coherence tomography (OCT) during pull back require a blood-free field of view for some seconds. Both saline and contrast fluids are used as blood substitutes in such applications. The analysis time is limited by the amount of blood substitute that can safely be applied. To improve performance and reduce contrast fluid or saline consumption, the intravascular device is suitably further configured as follows:
- the active member further comprises a reversibly deformable second active membrane that is arranged and configured to change its shape against the variable intravascular fluid pressure exerted on the second active membrane in an inserted state by the intravascular fluid;
- the pressure compensation cavity further comprises a third chamber, which is covered by the second active membrane and arranged between the first and second chambers and at a respective longitudinal distance therefrom along the longitudinal extension of the intravascular device; and
- the pressure compensation cavity further comprises a connection cavity that connects the first chamber, the second chamber and the third chamber and establishes the pressure communication between the first, second and third chambers.

Suitably, further, as described before in the context of other embodiments, the intravascular device of embodiments suitable for this kind of applications comprises a central lumen enclosed by a wall, the intravascular pressure compensation cavity being arranged in the wall of the intravascular device and not being in pressure communication with the central lumen. The central lumen allows receiving and guiding the optical instrumentation to the field of view located between the first and second active membranes.

Transport of contrast fluid through the intravascular device can be achieved through suitable lumens forming channels in the wall enclosing the central lumen, implemented in a way known per se. The contrast fluid can also be transported through the central lumen. In other embodiments, the contrast fluid can be transported through a tube in either the central lumen or the lumen in the wall enclosing the central lumen.

Contrast fluid can be ejected from the respective transport lumen through a hole or holes in the outer wall provided on the outer diameter. If a ring actuator and compensation membrane is used that covers the full outer diameter of the device, then the fluid can flow through the central lumen or a lumen(s) in the wall that lies for instance closer to the inner diameter than the pressure compensation cavity. If the active member and pressure compensation cavity only cover a part of the circumference, the contrast fluid can be supplied through lumens in the wall that lie in-between these.

It is noted that the use of a central lumen is not a requirement. Embodiments of the intravascular device in the form of a guidewire do not have a central lumen. For instance, an optical sensor for optical applications including an optical fiber for spectral imaging may be embedded in a guidewire that does not have a central lumen. Variants of such a guidewire embodiment have an active member in the form of an expandable balloon-like member to locally remove blood and thus improve spectral imaging.

This way, by expansion of both active membranes of the active member, an improved fluid containment within a longitudinal range limited by the first active membrane on one end and the second active membrane on the other end is achieved. Dilution of an image contrast agent dilution by blood can be reduced.

The optical sensor is in such embodiments preferably arranged and configured to emit the sensor light into the ambient outside the intravascular device and receive the scattered sensor light and from the ambient in a longitudinal ambient range between the longitudinal positions of the first active member and the second active member. Suitably, in such embodiments, the optical path from the central lumen to the outer ambient in the blood vessel is made using optically transparent materials. For instance, a transparent active material such as a transparent EAP is used for an actuator driving expansion and contraction of the active membrane. Also, any electrodes arranged in the optical path are preferably made using transparent materials, such as indium tin oxide (ITO), transparent conducting polymers and a transparent backing, for example polycarbonate. The pressure compensation cavity in the wall enclosing the central lumen is suitably filled with a transparent material, such as air, or with an index matched transparent fluid.

Summarizing the advantageous effects that can be achieved by this group of elements, a reduced pull back speed can be employed, improved images in OCT can be achieved, as well as an inspection of longer artery lengths, and additional optical sensing such as spectral techniques is enabled, which can be used to determine the composition of vulnerable plaque.

The intravascular device of the present invention comprises the blood pressure compensation cavity as a generic building block for endovascular applications. In other words, it is applicable in intravascular applications in general. Among the many different embodiments of the intravascular device that are provided for different application contexts is a large number of catheter types, such as a balloon catheter, a delivery catheter for stents or implants, a sensing catheter, for instance for optical sensing, in particular for application in hemodynamic sensing, or as an imaging catheter, to name only a limited number of catheter examples. Other embodiments of the intravascular device are provided in the form of a guide wire, a needle, or an endocavity device for intravascular insertion, or of an intravascular device for in-body tissue characterization, for instance an endoscope for optical sensing or imaging.

The intravascular device can be manufactured and delivered as such, in particular allowing a replacement of a used intravascular device.

A further embodiment that also forms an aspect of the present invention is intravascular system, which comprises the intravascular device of the present inventions or one of its embodiments, and which additionally comprises a control device for controlling operation of the active member of the intravascular device.

The control unit is in some embodiments provided with control features that further improve performance of the intravascular system. In some embodiments using an optical sensor in the intravascular device, the control unit receives sensor signals from the optical sensor providing for instance detected image information or detected spectral information. The control unit is preferably configured to evaluate the sensor signals, for instance to determine an extension of the vessel (such as an artery), or to detect a material composition using spectral information, which can be employed to detect a presence of plaque in the vessel. The evaluation is used in preferred embodiment to determine or adapt a control signal provided by the control unit to the active member for driving the active member, so that for instance the expansion of the active membrane can be adapted. This may be used, in embodiments having two active membranes as described before, to achieve an optimal enclosure of blood substitutes, in particular without making contact with the artery wall or plaque that has been detected. Contact with plaque can lead to high risk ruptures. In further embodiments, the control unit is configured to process received image information to determine and process image quality parameters and to provide a control signal for controlling active member expansion, thus forming a feedback control loop. The same control principles can be used for optical sensing and characterization applications.

It shall be understood that the intravascular device of claim 1 and the intravascular system of claim 14 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows a known intravascular device inserted inside a vessel;
Fig. 2 shows a schematic representation of a sectional view of an embodiment of an intravascular device inside a vessel;
Fig. 3 shows a schematic representation of a sectional view of another embodiment of an intravascular device inside a vessel;
Fig. 4 shows a schematic representation of a sectional view of a section of an intravascular device inside a vessel and connected to a control device, the intravascular device and the control device forming an embodiment of an intravascular system;
Fig. 5 shows a schematic representation of a sectional view of a section of another intravascular device inside a vessel and connected to another control device, the intravascular device and the control device forming another embodiment of an intravascular system;
Fig. 6 shows a schematic representation of a sectional view of another embodiment of an intravascular device inside a vessel and connected to another control device, the intravascular device and the control device forming another embodiment of an intravascular system;
Fig. 7 shows a schematic representation of a cross sectional view of another embodiment of an intravascular device inside a vessel, comprising an optical sensor and connected to another control device, the intravascular device and the control device forming another embodiment of an intravascular system;

### DETAILED DESCRIPTION OF EMBODIMENTS

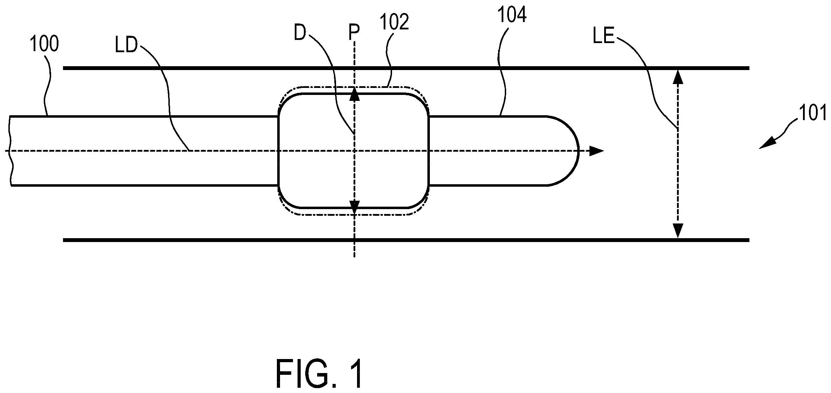

Fig. 1 shows an example of a known intravascular device 100, such a catheter or a guidewire, with an active member 102 for reversibly expanding a radial extension D of the intravascular device 100 inside a luminal extension LE of a vessel 101. The intravascular device 100 includes an outer wall 104 of the intravascular device. The active member 102 comprises is reversibly deformable. Inside the intravascular device 100, a pressure value in a central lumen equals an atmospheric pressure value, since the central lumen is in fluid communication with a region outside the intravascular device that is also at the atmospheric pressure value. When the intravascular device 100 is inserted into e.g. a blood vessel, the active member 102, in order to expand, has first to overcome the time-varying blood pressure acting against the expansion and to generate an expansion force necessary for the expansion. In particular, the pressure variation between systolic and diastolic pressure results in a change of shape of the active member 102. The pressure variation may also cause vibration of the active member or a variation in an expansion force applied against the blood pressure. Therefore, in such a typical intravascular device, the expansion force varies with the varying current blood pressure.

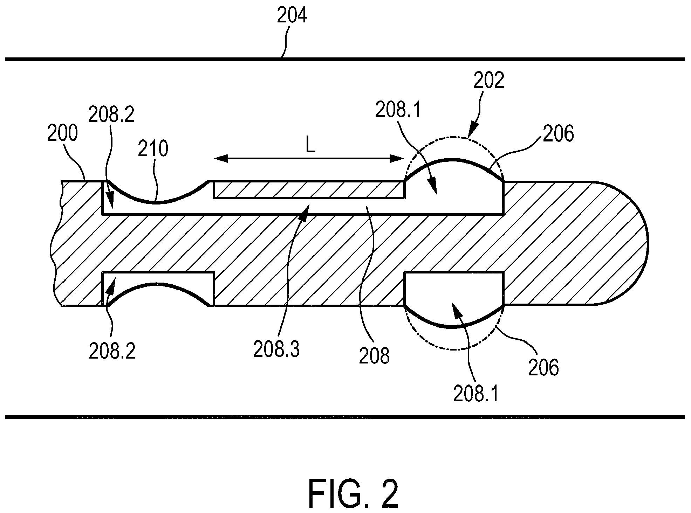

Fig. 2 shows a schematic representation of a sectional view of an embodiment of an intravascular device 200 according to the present invention. The intravascular device 200 comprises an active member 202 for reversibly expanding a radial extension of the intravascular device inside a luminal extension of a vessel 204 that is filled with an intravascular fluid (not shown) that exerts pressure on the intravascular device 200. The active member 202 comprises a reversibly deformable active membrane 206 that is arranged and configured to actively change its shape against a variable intravascular fluid pressure exerted on the active membrane 206 in an inserted state by an intravascular fluid. Fig. 2 shows the active membrane 206 in two different states, represented by a solid and a dotted line, respectively. The two different states represent two different radial extensions of the intravascular device 200, which are effected by the active member 202.

The intravascular device 200 also comprises an intravascular pressure compensation cavity 208. The intravascular pressure compensation cavity 208 includes a first chamber 208.1 that is covered by the active membrane 206, a second chamber 208.2 that is covered by a compensation membrane 210, and a connection cavity 208.3 that extends from the first chamber 208.1 to the second chamber 208.2. The first and second chambers of the intravascular device 200 are arranged at a longitudinal distance L from each other along a longitudinal extension of the intravascular device. The compensation membrane 210 is also reversibly deformable in response to the variable intravascular fluid pressure.

The intravascular pressure compensation cavity 208 is fluidicly sealed from the intravascular fluid by the active membrane 206 and by the compensation membrane 210.

Thus, by means of the connection cavity 208.3 that extends from the first chamber 208.1 to the second chamber 208.2 pressure communication between the first and the second chamber the first chamber 208.1 and the second chamber 208.2 is established. Furthermore, the intravascular pressure compensation cavity 208 is also in pressure communication with the intravascular fluid via the active membrane 206 and the compensation membrane 210. Therefore, the intravascular fluid pressure is also acting on an inner side of the active membrane 206, which inner side faces the first chamber 208.1. In effect, the shape of the active membrane of the active member is stabilized by the pressure compensation cavity 208, thus preventing large shape fluctuations as a result of intravascular fluid pressure variation. Also, the force generated for expanding the radial extension of the intravascular device against the intravascular fluid pressure is increased.

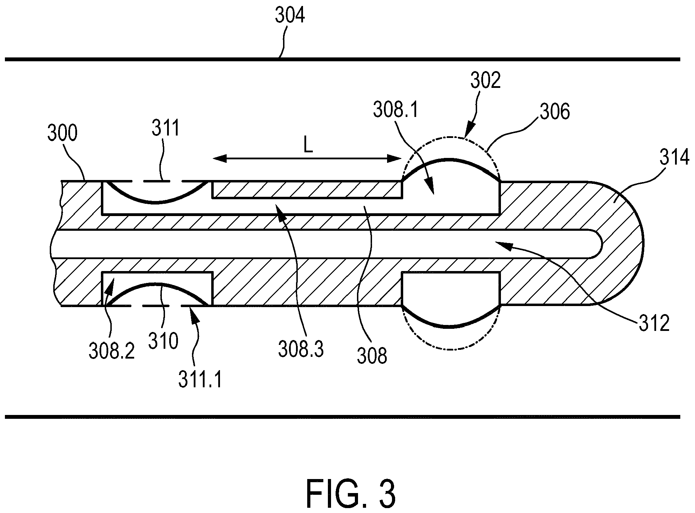

Fig. 3 shows a sectional view of another embodiment of an intravascular device 300 inserted in a vessel 304. Reference numerals of structural elements of the intravascular device 300 that correspond to structural elements of the intravascular device 200 of Fig. 2 differ only in their first digit, which is "3" instead of "2". Therefore, for brevity of the present description, reference is also made to the description of Fig. 2 for details of these structural elements. The following description mainly concentrates on features distinguishing the two embodiments from each other.

Thus, the intravascular device 300 comprises an active member 302 with an active membrane 306, and an intravascular pressure compensation cavity 308. In the present embodiment, the second chamber 308.2 of the intravascular pressure compensation cavity 308 is partially covered by a stiff protective cover piece 311 that is arranged between the compensation membrane 310 and the intravascular fluid (not shown) inside the vessel 304. The stiff protective cover piece 311 contains openings 311.1 for allowing pressure communication between the intravascular fluid and the compensation membrane 310. In the schematic representation of Fig. 3, two such openings are shown. However, this is not meant as indicating a necessary requirement. Any number and size of openings allowing pressure communication through the stiff protective cover piece 311 can be used. Similarly, the intravascular device 200 described with respect to Fig. 2 may further comprise a stiff protective cover piece for protection of the compensation membrane, analogous to the protective cover piece 311 described here.

The intravascular device 300 further comprises a central lumen 312 enclosed by a wall 314. In the intravascular device 300, the intravascular pressure compensation cavity 308 is arranged in the wall 314 and is not in pressure communication with the central lumen 312.

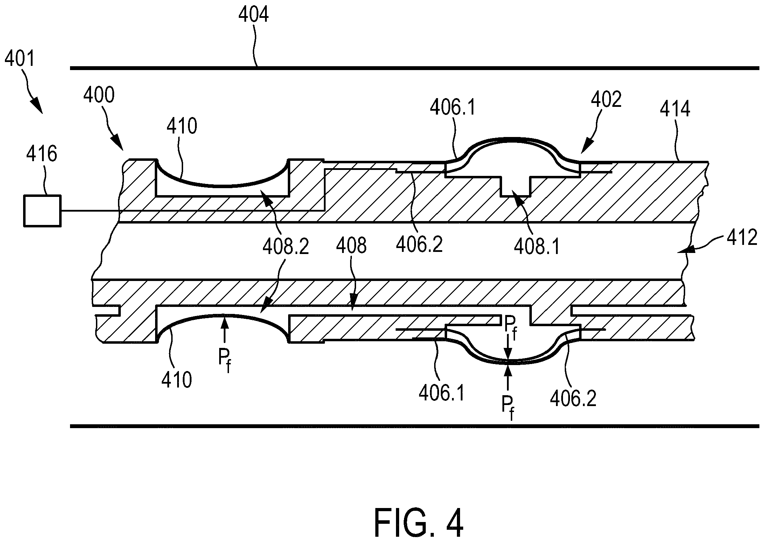

Fig. 4 shows a sectional view of an intravascular device 400. The figure also shows an embodiment of an intravascular system 401.

Reference numerals of structural elements of the intravascular device 400 that correspond to structural elements of the intravascular device 200 or 300 of Figs. 2 and 3 differ only in their first digit, which is "4" instead of "2" or "3". Therefore, for brevity of the present description, reference is also made to the description of Figs. 2 and 3 for details of these structural elements. The following description mainly concentrates on features distinguishing the embodiment of Fig. 4 from those of Figs. 2 and 3.

Only a longitudinal portion of the intravascular device is shown. As before, the intravascular device 400 is shown in an inserted state inside a vessel 404. The intravascular device 400 comprises an active member 402 having a reversibly deformable active membrane 406.1 that is arranged and configured to change its shape against a variable intravascular fluid pressure P_{f} exerted on the active membrane 406.1 in an inserted state by an intravascular fluid that fills the vessel 404. The active member 402 also comprises an actuator that is configured to receive a first control signal from an external control device 416 for controlling the operation of the active member 402. In the present embodiment, the actuator is formed by an electroactive polymer (EAP) layer 406.2 that receives the first control signal and is in mechanical contact with the active membrane 406.1. The EAP layer 406.2 is configured to controllably change its layer shape in accordance with the received first control signal. Electroactive polymers (EAPs) and their advantages have been described in more detail hereinabove. In short, EAPs are polymers that exhibit a change in size or shape when stimulated by an electric field.

As in the previous embodiments, a pressure compensation cavity 408 comprises a compensation membrane 410 that covers the second chamber 408.2. Both the first and the second chambers 408.1 and 408.2 are in pressure communication with each other and, via the active membrane 406.1, the EAP layer 406.2 and the compensation membrane 410, with the intravascular fluid filling the vessel 404. This achieves that the intravascular fluid pressure P_{f} is exerted from two sides on the active member 402, in particular on the active membrane 406.1 and the EAP layer 406.2, thus achieving pressure compensation.

Suitable materials for the active membrane 406.1 and for the compensation membrane 410 are thermoplastic polymers, such as a polyether block amide, for instance known under the trade name Pebax. Other suitable materials include polyethylene terephthalate (PET), polyurethane, silicones, blends of the mentioned materials, multilayer structures made of the mentioned materials.

The intravascular device 400 also comprises a central lumen 412 enclosed by a wall 414.

The intravascular device 400 and the control device 416 form an embodiment of an intravascular system 401.

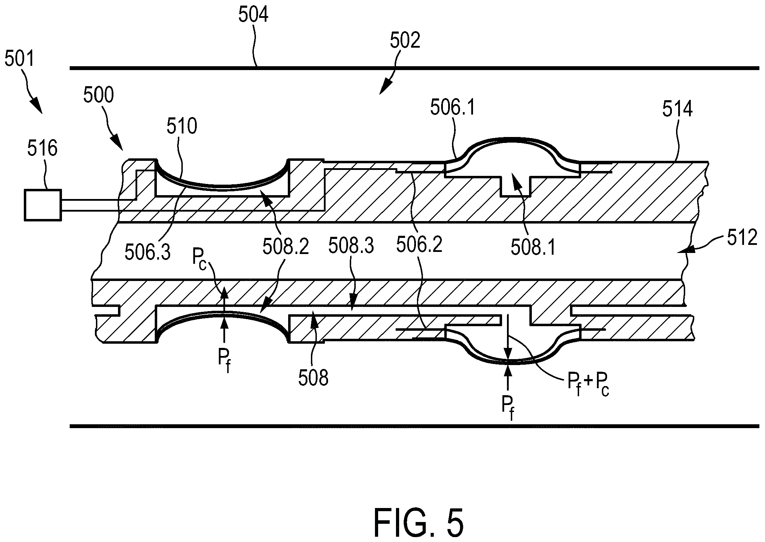

Fig. 5 shows a sectional view of a portion of an intravascular device 500.

Reference numerals of structural elements of the intravascular device 500 that correspond to structural elements of the intravascular device 400 of Fig 4 differ only in their first digit, which is "5" instead of "4". Therefore, for brevity of the present description, reference is also made to the description of Fig 4 and the other preceding Figures for details of these structural elements. The following description mainly concentrates on features distinguishing the embodiment of Fig. 4 and 5 from each other.

As before, the intravascular device 500 is shown in an inserted state inside a vessel 504 and only a longitudinal portion of the intravascular device 500 is shown.

The active member 502 of the intravascular device further comprises a compensation actuator 506.3 that is configured to receive a compensation control signal from a control device 516, for controlling operation of the compensation actuator 506.3 and to drive a deformation of the compensation membrane 510 in accordance with the received compensation control signal. The compensation actuator 506.3 is formed by a second electroactive polymer layer that is in mechanical contact with the compensation membrane 510 and that is configured to controllably change its layer shape in accordance with the compensation control signal delivered by the control device 516.

By driving a contraction of the compensation membrane 510 by an additional force Pc, and using the pressure communication effected by the pressure compensation cavity 508, a total pressure of P_{f}+P_{C} is exerted from the inner side of the first chamber 508.1 to the active membrane 506.1, in absence of a driving force exerted by the actuator 506.2. When additionally providing an actuation force generated by the actuator 506.2, the total force available for expansion of the active membrane 506.1 is increased over that provided in the embodiment of Fig. 4. Thus, using the compensation actuator 506.3 in combination with an expansion of the active membrane 506.1 driven by the actuator 506.2 it is possible to further enhance expansion and generation of force by the active membrane 506.1 against the intravascular fluid pressure.

The intravascular device 500 and the control device 516 form another embodiment of an intravascular system 501.

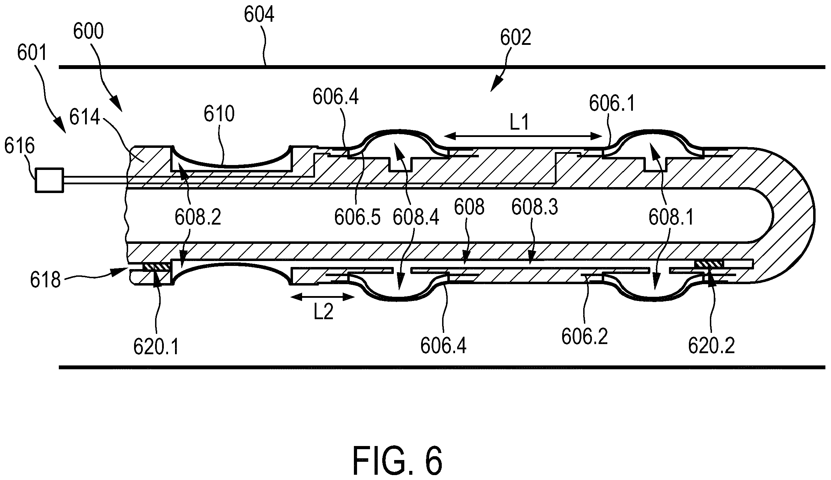

Fig. 6 shows a sectional view of an embodiment of an intravascular device 600 inserted inside a vessel 604.

Reference numerals of structural elements of the intravascular device 600 that correspond to structural elements of the intravascular device 400 of Fig 4 differ only in their first digit, which is "6" instead of "4". Therefore, for brevity of the present description, reference is also made to the description of Fig 4 and the other preceding Figures for details of these structural elements. The following description mainly concentrates on features distinguishing the embodiment of Fig. 4 and 6 from each other.

The active member 602 of the intravascular devices comprises a reversibly deformable active membrane 606.1 driven by an actuator comprising an EAP layer 606.2, and a reversibly deformable second active membrane 606.4, which is driven by a second EAP layer 606.5 to change its shape against the variable intravascular fluid pressure exerted on the second active membrane in an inserted state by the intravascular fluid. To include the second active membrane into the pressure compensation structure of the present invention, the pressure compensation cavity 608 of the intravascular device 600 comprises a first chamber 608.1 covered by the active membrane 606.1, a second chamber 608.2 covered by a compensation membrane 610, and a third chamber 608.4 that is covered by the second active membrane 606.4. The third chamber 608.4 is arranged between the first and second chambers 608.1 and 608.2, at respective longitudinal distances L1 and L2 therefrom. The pressure compensation cavity 608 also comprises a connection cavity 608.3 that connects the first chamber 608.1, the second chamber 608.2 and the third chamber 608.4 and establishes the pressure communication between these chambers.

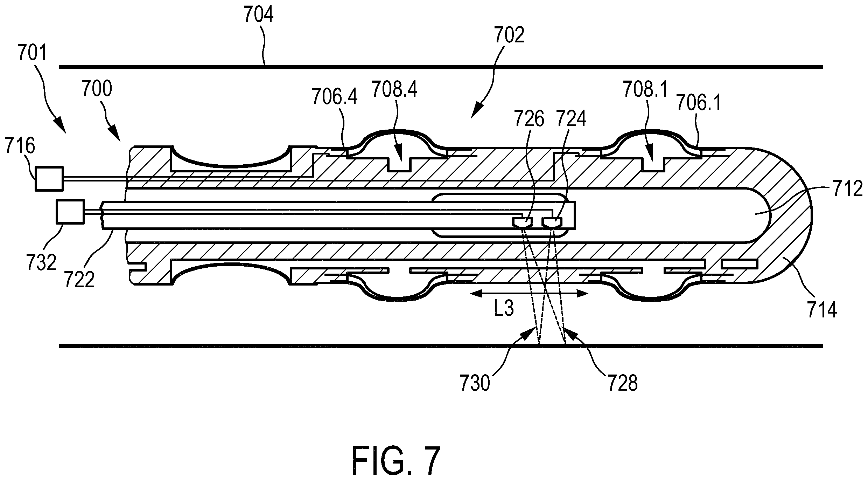

In operation, the first and second active membranes 606.1 and 606.4 are driven in parallel to achieve an enclosure of longitudinal extension L1 between them. This can be used to reduce or fully avoid dilution of a contrast fluid. A channel for transporting the contrast fluid along the intravascular device 600 is not shown in Fig. 6 for simplicity of graphical representation. Such channels, their fabrication and arrangement are known per se in the art. An application case for this intravascular device 600 will be described below with reference to Fig. 7.

In the following, the fabrication of the pressure compensation cavity 608 of the intravascular device 600 is described. The process is similarly applicable to the embodiments of the Figures 2 to 5 and 7.

In the intravascular device 600 the connection cavity 608.3 is formed by sealing an open lumen 618 that is embedded in a wall 614 of the intravascular device 600. The sealing is performed in this particular case by two sealing elements 620.1 and 620.2 forming the longitudinal end points of the connection cavity 608.3 of the intravascular device. In other words, the sealing elements 620.1 and 620.2 are arranged so that the first chamber 608.1, the second chamber 602.3 and the third chamber 608.4 are arranged between them. The sealing elements 620.1 and 620.2 are advantageously implemented using for instance a gluing or a thermal sealing process.

The intravascular device 600 and the control device 616 form another embodiment of an intravascular system 601

Fig. 7 shows a sectional view of an embodiment of an intravascular device 700. Reference numerals of structural elements of the intravascular device 700 that correspond to structural elements of the intravascular device 600 of Fig 6 differ only in their first digit, which is "7" instead of "6". Therefore, for brevity of the present description, reference is also made to the description of Fig 6 and the other preceding Figures for details of these structural elements. The following description mainly concentrates on features distinguishing the embodiment of Fig. 6 and 7 from each other.

The wall 714 of the intravascular device 700 is configured to allow transmission of electromagnetic radiation in a predetermined spectral sensing range. Furthermore, an optical sensor 722 is arranged in a central lumen 712. The optical sensor 722 comprises a light emitting unit 724 configured to emit sensor light 728 in the spectral sensing range through the wall 714. It also comprises a light sensor 726 configured to receive scattered sensor light 730 that has been scattered by an ambient of the intravascular device. In particular, the optical sensor 722 is arranged and configured to emit the sensor light into the ambient and receive the scattered sensor light and from the ambient in a longitudinal ambient range L3 between the longitudinal positions of the first chamber 708.1 and the third chamber 708.4.

The optical sensor 722 is connected to a processing unit 732 that controls the emission of the sensor light by the light emitting unit 724 and processes a sensor signal indicative of the scattered sensor light received by the light sensor 726. This exemplary intravascular device 700 is advantageously configured to be used for contrast fluid reduction in optical sensing or imaging. If the intravascular device is inserted into a blood vessel, blood, being thus the intravascular fluid, strongly scatters light emitted by the optical sensor 722. Techniques such as spectral analysis of the blood vessel wall or plaque, or optical coherence tomography (OCT) require a blood low or even blood free ambient for a given amount of time. In this case usually saline or contrast fluids are used and a maximum analysis time depends on the amount of saline or contrast fluid that can be applied. By using the control device 716 to drive an expansion of the active membranes 706.1 and 706.4 (e.g. simultaneously) an outer diameter of the intravascular sensor is enlarged so that blood mixing with the saline or contrast fluid that is present or is injected in a region of the vessel located between both active membranes is reduced and a SNR ratio of the sensor signal is improved.

In other embodiments of an intravascular device it is possible to emit the sensor light into the ambient and receive the scattered sensor light and from the ambient through an active member that comprises a transparent (at least within the spectral sensing range) layer, such as for example ITO or transparent conducting polymers, and a transparent active membrane comprising for example a polycarbonate layer

The pressure compensation cavities of the intravascular devices described with reference to Figs. 2-8 may be filled with a gas, a liquid or a viscoelastic material, depending on the requirements of the intravascular device.

The size of the pressure compensation membrane surface area may be close to the size of the surface area of the actuator. In other embodiments, it is smaller in size than the surface area of the actuator. In yet another embodiment, it is larger in size in comparison with the surface area of the actuator.

The intravascular devices described with reference to Figs. 2-8 may form a catheter, such as a balloon catheter, a delivery catheter (stents, implants), a sensing catheter (optical, in particular for hemodynamic sensing), an imaging catheter,. They may alternatively form a guide wire, a needle, or an endocavity device for intravascular insertion.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

Any reference signs in the claims should not be construed as limiting the scope.

## Claims

1. An intravascular device (200) for insertion into a vessel of a living being (204), comprising:
- an active member (202) for reversibly expanding a radial extension of the intravascular device inside a luminal extension of the vessel and comprising a reversibly deformable active membrane (206) that is arranged and configured to change its shape against a variable intravascular fluid pressure exerted on the active membrane in an inserted state by an intravascular fluid;
- an intravascular pressure compensation cavity (208), which is fluidicly sealed from the intravascular fluid and which includes:
- a first chamber (208.1) that is covered by the active membrane (206), and
- a second chamber (208.2) that is covered by a compensation membrane (210), which is reversibly deformable in response to the variable intravascular fluid pressure, wherein
- the first chamber (208.1) and the second chamber (208.2) are in pressure communication with each other and, via the active membrane (206) and the compensation membrane (210), with the intravascular fluid.

2. The intravascular device of claim 1, wherein
- the first chamber (208.1) and the second chamber (208.2) are arranged at a longitudinal distance (L) from each other along a longitudinal extension of the intravascular device; and wherein
- the pressure compensation cavity (208) further comprises a connection cavity (208.3) that extends from the first chamber to the second chamber and establishes the pressure communication between the first and the second chamber.

3. The intravascular device of claim 1, wherein intravascular device comprises a central lumen (312) enclosed by a wall (314), and wherein the intravascular pressure compensation cavity (308) is arranged in the wall of the intravascular device and is not in pressure communication with the central lumen.

4. The intravascular device of claim 1, wherein the pressure compensation cavity is filled with a gas, a liquid or a viscoelastic material.

5. The intravascular device of claim 1, comprising a stiff protective cover piece (311) that is arranged between the compensation membrane and the intravascular fluid and that contains at least one opening (311.1) for allowing pressure communication between the intravascular fluid and the compensation membrane.

6. The intravascular device of claim 1, wherein the active member (402) comprises an actuator (406.2) that is configured to receive a first control signal and to drive an expansion or contraction of the active membrane in accordance with the received first control signal.

7. The intravascular device of claim 6, wherein the actuator (406.2) comprises an electroactive polymer layer that is in mechanical contact with the active membrane (406.1) and that is configured to controllably change its layer shape in accordance with the received first control signal.

8. The intravascular device of claim 1, wherein the active member further comprises a compensation actuator (506.3) that is configured to receive a compensation control signal and to drive a deformation of the compensation membrane in accordance with the received compensation control signal.

9. The intravascular device of claim 8, wherein the compensation actuator (506.3) comprises a second electroactive polymer layer that is in mechanical contact with the compensation membrane and that is configured to controllably change its layer shape in accordance with the compensation control signal.

10. The intravascular device of claim 2 and 3, wherein
- the active member (602) further comprises a reversibly deformable second active membrane (606.4) that is arranged and configured to change its shape against the variable intravascular fluid pressure exerted on the second active membrane (606.4) in an inserted state by the intravascular fluid;
- the pressure compensation cavity further comprises a third chamber (608.4), which is covered by the second active membrane (606.4) and arranged between the first (608.1) and second chambers (608.2) and at respective longitudinal distances (L1, L2) therefrom along the longitudinal extension of the intravascular device; and wherein
- the pressure compensation cavity (608) further comprises a connection cavity (608.3) that connects the first chamber, the second chamber and the third chamber and establishes the pressure communication between the first, second and third chambers.

11. The intravascular device of claim 3, wherein
- the wall (714) of the intravascular device (700) is configured to allow transmission of electromagnetic radiation in a predetermined spectral sensing range;
- an optical sensor (722) is arranged in the central lumen (712) and configured to emit sensor light (728) in the spectral sensing range through the wall and receive scattered sensor light (730) that has been scattered by an ambient of the intravascular device.

12. The intravascular device of claims 9 and 11, wherein the optical sensor (722) is arranged and configured to emit the sensor light into the ambient and receive the scattered sensor light and from the ambient in a longitudinal ambient range (L3) between the longitudinal positions of the first active member and the second active member.

13. The intravascular device of claim 1, which forms a catheter, a guide wire, a needle, or an endocavity device for intravascular insertion.

14. An intravascular system (401), comprising:
- the intravascular device of claim 1; and
- a control device (416) for controlling operation of the active member.
